# EUROPÄISCHE PATENTSCHRIFT

(11) **EP 3 097 787 B1**
(45) Veröffentlichungstag und Bekanntmachung des Hinweises auf die Patenterteilung: **21.11.2018**
(21) Anmeldenummer: 15169287.8
(22) Anmeldetag: 26.05.2015
(51) Int. Cl.: A23J 1/20, C07K 14/79

(54) **VERFAHREN ZUR GEWINNUNG VON LACTOPEROXIDASE AUS MILCH**
PROCESS FOR OBTAINING LACTOPEROXIDASE FROM MILK
PROCÉDÉ POUR OBTENIR LACTOPEROXIDASE À PARTIR DU LAIT

(30) Priorität: 24.05.2015 EP 15169063
(43) Veröffentlichungstag der Anmeldung: 30.11.2016
(73) Patentinhaber: DMK Deutsches Milchkontor GmbH, 27404 Zeven (DE)
(72) Erfinder: Aschemann, Jan, 27404 Holzminden (DE); Döring, Sven-Rainer, 27404 Holzminden (DE)
(74) Vertreter: Fabry, Bernd

(56) Entgegenhaltungen:
- WO-A1-93/13676
- WO-A1-2015/009400
- QIAO-YAN DU ET AL: "An integrated expanded bed adsorption process for lactoferrin and immunoglobulin G purification from crude sweet whey", JOURNAL OF CHROMATOGRAPHY B, Bd. 947-948, 1. Februar 2014 (2014-02-01), Seiten 201-207, XP055138174, ISSN: 1570-0232, DOI: 10.1016/j.jchromb.2013.12.020

## Beschreibung

Die Erfindung befindet sich auf dem Gebiet der Nahrungsmittelindustrie und betrifft ein Verfahren zur Gewinnung von Lactoperoxidase aus Rohmilch.

### STAND DER TECHNIK

Das Enzym Lactoperoxidase (LPO) kommt in den meisten Tieren vor und wird im Menschen von den Brustdrüsen, Speicheldrüsen und Schleimdrüsen der Bronchien ausgeschieden. Es katalysiert die Oxidation von Phenolen und verschiedenen Anionen durch Wasserstoffperoxid. Die Reaktionsprodukte sind hochreaktive Moleküle, die toxisch auf in den Körper eingedrungene Mikroorganismen wirken. LPO ist daher ein Teil des angeborenen (unspezifischen) Immunsystems und ermöglicht die Neutralisierung von Bakterien in der Milch und anderen Schleimhautsekreten. Die Struktur der Lactoperoxidase besteht hauptsächlich aus α-Helices. Hinzu kommen zwei kurze antiparallele β-Faltblätter. Ein Häm-Cofaktor befindet sich nahe dem Zentrum des Apoproteins. Die Lactoperoxidase katalysiert die Oxidation verschiedener Sauerstoff-Akzeptoren durch Wasserstoffperoxid:

(1) reduzierter Akzeptor + H₂O₂ → oxidierter Akzeptor + H₂O

Beispiele solcher Oxidationsreaktionen sind:

(2) Thiocyanat (SCN⁻) → Hypothiocyanit (OSCN⁻)

(3) Bromid (Br⁻) → Hypobromit (BrO⁻)

(4) lodid (I⁻) → Hypoiodit (IO⁻)

Quelle des Wasserstoffperoxids ist vielfach die Reaktion von Glucose mit Sauerstoff in Gegenwart des Enzyms Glucose-Oxidase (EC 1.1.3.4), die ebenfalls im Speichel stattfindet. Die Glucose wiederum kann in Gegenwart des Speichel-Enzyms Amyloglucosidase (γ-Amylase (EC 3.2.1.3)) aus Stärke entstehen. Derartige Oxidationsprodukte sind hoch reaktiv und antibakteriell stark wirksam. Das Lactoperoxidase-System ist in der Lage, eine ganze Reihe von aerobischen und anaerobischen Bakterien, darunter auch mikroaerophile Helicobacter pylori. Der Effekt des Lactoperoxidase-Systems hängt von bestimmten experimentellen Bedingungen ab. Wenn Bakterien unter aerobischen Bedingungen auf Nähragar kultiviert werden sollen, nachdem sie dem Lactoperoxidase-System ausgesetzt waren, wachsen sie nicht. Allerdings wachsen sie sehr wohl auf Blut-Agar unter anaerobischen Bedingungen. Das Lactoperoxidase-System scheint synergistisch mit Lactoferrin und Lysozym zu agieren. Ist Wasserstoffperoxid im Überschuss zum Thiocyanat vorhanden, kann das Lactoperoxidase-System auch cytotoxisch wirken. Aufgrund der antibakteriellen Wirksamkeit des Lactoperoxidase-Systems wird es zur Konservierung von Nahrungsmitteln und Kosmetika sowie in der Augenheilkunde eingesetzt. Weitere Anwendungen finden sich in den Bereichen Zahnheilkunde und Wundbehandlung. Möglicherweise kann das Lactoperoxidase-System auch zur Bekämpfung von Tumoren und Viren eingesetzt werden. Das Lactoperoxidase-System hemmt das Wachstum der bakteriellen Flora in der Milch und in Milchprodukten. Die Zugabe von Wasserstoffperoxid und einem Thiocyanat verlängert die Haltbarkeit gekühlter Rohmilch. Das Lactoperoxidase-System ist vergleichsweise wärmestabil und wird als Indikator für eine Überpasteurisierung von Milch verwendet. Das Lactoperoxidase-System scheint auch zur Behandlung von Karies, Gingivitis und Parodontitis geeignet zu sein und wird daher als Bestandteil von Zahnpasten und Mundspüllösungen eingesetzt. Weil es das Bakterienwachstum in der Mundhöhle hemmt, hemmt es auch die Säureproduktion dieser Bakterien.

Lactoperoxidase ist damit ein sehr interessanter natürlicher Wirkstoff, lässt sich bislang nur mit hohem Aufwand in ausreichender Reinheit aus speziellen Milchfraktionen mit Hilfe von chromatographischen Methoden gewinnen. So beschreibt beispielsweise EP 1401289 B1 (UPFRONT) ein Verfahren zur Gewinnung von Lactoperoxidase aus Magermilch, aber auch Süßmolke und anderen Milchbestandteilen, unter Einsatz der EBA (Expanded Bed Adsorption), bei der pro Liter Einsatzstoff 54 mg Produkt unbekannter Reinheit erhalten werden. Im Hinblick auf den kommerziellen Wert des Produktes auf der einen Seite und der geringen Konzentrationen, in denen es zum anderen in der Milch enthalten ist, besteht ein hohes Interesse daran, die Ausbeuten zu verbessern.

Eine Aufreinigung von Lactoperoxidase mit Hilfe der Hydrohobic Interaction Chromatography wird in CN 103709246 A (BEIJING BIOTECHNOLOGY) beschrieben, ist aber nur für den Labormaßstab zu realisieren, da eine Produktion viel zu teuer wäre.

Die Aufgabe der Erfindung hat somit darin bestanden, ein für die kommerzielle Herstellung geeignetes Verfahren zu entwickeln, dass den oben beschriebenen Anforderungen Rechnung trägt und Lactoperoxidase aus Milch in vergleichsweise höheren Ausbeuten und höherer Reinheit bei geringst möglichem technischen Aufwand zur Verfügung stellt

### BESCHREIBUNG DER ERFINDUNG

Gegenstand der Erfindung Verfahren zur Gewinnung von Lactoperoxidase, bei dem man
(a) Rohmilch einer Kaltseparation bei einer Temperatur von etwa 8 bis etwa 15 °C unterwirft und den Rahm abtrennt,
(b) die kalt separierte Magermilch einer Expanded Bed Adsorption (EBA) unterwirft, wobei die Lactperoxidase vom Adsorbens in der EBA-Säule adsorbiert wird, und anschließend
(c) die Lactoperoxidase wieder eluiert, entsalzt, entwässert und als trockenes Produkt isoliert.

Überraschenderweise wurde gefunden, dass die Kombination von Kaltseparation und EBA ein geeignetes Verfahren darstellt um ausgehend von Rohmilch mit dem höchsten Gehalt an Lactoperoxidase innerhalb der Gruppe der Milchfraktionen Lactoperoxidase mit geringem technischen Aufwand in hohen Ausbeuten und hoher Reinheit zu gewinnen. Durch geeignete Auswahl des Eluenten in der EBA können Ausbeute und Reinheit weiter gesteigert werden.

### KALTSEPARATION

Im erfindungsgemäßen Verfahren ist vorgesehen, dass die Temperatur des kalten Zustandes der Rohmilch durch Wärmeaustausch mit einem Wärmeträgermedium auf einen für ihre Separation optimalen Wert eingestellt wird. Normalerweise steht die Rohmilch in einem gekühlten Zustand zur Verfügung, dessen Temperatur nicht dem Wert entspricht, bei dem ihre Kaltseparation am wirkungsvollsten und für das Milchfett (Rahm) am schonendsten betrieben werden kann. Deshalb wird sie durch den Wärmeaustausch auf den für ihre Separation optimalen Wert eingestellt. Die dabei anfallende Austauschkälte kann insbesondere durch eine sogenannte Wärmeschaukel anderen Prozessen, die innerhalb eines Molkereibetriebs ablaufen, zur Verfügung gestellt werden. Beispielsweise ist die Temperatur der gekühlten Rohmilch nicht höher als 6 °C, während die für die Kaltseparation optimale Temperatur im Bereich von 8 bis 15 °C und insbesondere 8 bis 12 °C liegt. In diesem Fall erfolgt der Wärmeaustausch durch Anwärmen der Rohmilch, damit die Temperatur ihres kalten Zustandes auf einen in diesem Bereich liegenden Wert angehoben wird. Normalerweise liegt in Molkereibetrieben ein Wärmeüberschuss vor. Deshalb kann als Wärmeträgermedium für das Anwärmen Niedrigtemperaturwasser, das in Prozessen des Molkereibetriebes anfällt, verwendet werden. Dieses Niedrigtemperaturwasser wird dem Wärmeaustauschprozess mit einer Temperatur, die beispielsweise im Bereich von 35 °C liegt, zugeführt und kühlt sich durch den Wärmeaustausch auf eine Temperatur, die beispielsweise im Bereich von 11 bis 15 °C liegt, ab. Dadurch stellt das erfindungsgemäße Verfahren eine wichtige Kältequelle für Prozesse des Molkereibetriebs zur Verfügung.

Die Abtrennung von Feststoffen sowie die Entrahmung des Fettanteils von etwa 4 Gew.-% erfolgen üblicherweise in einem nachgeschalteten Bauteil, vorzugsweise einem Separator. Derartige Bauteile sind aus dem Stand der Technik hinreichend bekannt. In der Milchindustrie sehr verbreitet sind Separatoren der Firma GEA Westfalia Separator GmbH, mit denen die Feststoffabtrennung einzeln oder gemeinsam durchgeführt werden können (http://www.westfalia-separator.com/de/anwendungen/molkereitechnik/milchmolke.html). Bevorzugte Kaltmilchseparatoren werden von diesem Hersteller unter der Bezeichnung "Procool" angeboten. Entsprechende Bauteile sind beispielsweise auch in der DE 10036085 C1 und der DE 10361526 B3 (Westfalia) beschrieben und dem Fachmann bestens bekannt, so dass es zur Durchführung dieser Verfahrensschritte keiner Erläuterungen bedarf, da sie den allgemeinen Fachwissen zuzurechnen sind.

### EXPANDED BED ADSORPTION

Die Prinzipien, nach denen Proteine gemäß der Expanded Bed Adsorption (EBA) gebunden werden, sind grundsätzlich von denen einer klassischen Säulenchromatographie, oder lonenaustauschertechnologie nicht verschieden. Wo die klassische Säulenchromatographie jedoch eine feste Phase in Form einer Packung einsetzt, nutzt die EBA die Partikel in einem fluidisierten Zustand, d.h. in einer Art Wirbelschicht, wobei sich das Volumen idealerweise um den Faktor 2 ausdehnt. Wesentlich ist ferner, dass die Partikel unterschiedliche Größen und Dichten aufweisen, wodurch bei der Expansion des Bettes ein Partikelgrößengradient entsteht. In der Wirbelschicht entstehen dabei zusätzlich lokale Turbulenzen. Dies führt dazu dass insbesondere Bestandteile, die eine bestimmte Größe überschreiten oder die Tendenz aufweisen, zu agglomerieren, die Wirbelschicht durchströmen, was in einer festen Packung nicht möglich wäre.

Bei der EBA werden die Säulen, bei denen es sich vorzugsweise um Feststoff-Adsorptionssäulen handelt, von oben mit dem Adsorbens befüllt. Bei diesen handelt es sich zum einen um ein nicht-durchlässiges Material hoher Dichte und zum anderen um ein zur Proteinbindung befähigtes lonenaustauscherharz. Vorzugsweise weist das nicht-durchlässige Material eine Dichte von mindestens 1,5 g/ml und/oder eine Teilchengröße von höchstens 150 µm und insbesondere 40 bis 120 µm auf. Gängige Beispiele hierfür bilden Glas- oder Metallperlen, speziell Wolfram, wobei diese wie eingangs beschrieben unterschiedliche Durchmesser und Dichten aufweisen sollten, damit in der Wirbelschicht ein entsprechender Gradient entsteht. Dieses Material stellt den Träger in der Wirbelschicht dar.

Der zweite Teil des Adsorbers ist ein lonenaustauscherharz, das zur Bindung von Proteinen befähigt ist. Beispielsweise kommt hierfür ein Sulfonsäure-Anionenaustauscherharz auf Polysaccharidbasis in Betracht, wie es in der EP 1401289 B1 (Beispiel 3) beschrieben wird. Bevorzugt ist ein Wolframträger, der mit dem Harz beschichtet wird.

Grundsätzlich können Träger und Ionenaustauscher nacheinander oder gemeinsam in die Säule gefüllt werden. Es ist auch möglich, die Träger mit den Harzen zu beschichten. Nach dem Befüllen bilden die Trägermaterialien eine dichte Schüttung, die großen Aggregaten wenig Raum zum durchkommen bietet. Sobald das Bett in fluidisierten Zustand versetzt wird, beispielsweise dadurch, dass die Schüttung mit einem Magnetrührer aufgewirbelt wird, und die Sinkgeschwindigkeit der Aufwärtsströmung entspricht, bilden die Träger einen stabilen Konzentrationsgradienten aus. Ziel ist es, einen Flux einzustellen, bei dem die Geschwindigkeit der zu trennenden Stoffe größer ist als die der Trägerteilchen. Um den Unterschied in den Steiggeschwindigkeiten zu erhöhen, werden die Träger auch oftmals mit einem Quarz- oder Metallkern versehen.

Um Verunreinigungen zu entfernen hat es sich als vorteilhaft erwiesen, dass man die EBA-Säule vor und nach der Beladung mit der kalt separierten Magermilch equilibriert, beispielsweise mit Wasser oder einer Pufferlösung. Weitere vorteilhafte Ausgestaltungsformen dieses Verfahrensschrittes bestehen darin, dass man
- die kalt separierte Magermilch mit einer Fluxrate von etwa 3 bis etwa 50 cm/min aufbringt; und/oder
- das nicht-durchlässige Material hoher Dichte und das zur Proteinbindung befähigten lonenaustauscherharz im Gewichtsverhältnis von etwa 20:80 bis etwa 50:50 einsetzt; und/oder
- das Adsorbens bestehend aus dem nicht-durchlässigen Material hoher Dichte und dem zur Proteinbindung befähigten lonenaustauscherharz relativ zu dem auf die EBA-Säule zu ladenden kalt separierten Magermilch in einem v/v-Verhältnis von etwa 1:100 bis etwa 1:1.000 und vorzugsweise etwa 1:250 bis etwa 1:500 einsetzt.

Nachdem die Aufgabe vollständig ist, wird der fluidisierte Zustand beendet und der Packung Gelegenheit gegeben, sich wieder zu setzen. Dann erfolgt das pülen der Säule mit Wasser und die eigentliche Desorption der Lactoperoxidase vorzugsweise in Gegenstromrichtung, d.h. von unten nach oben, wobei als Eluenten vorzugsweise verdünnte Basen eingesetzt werden. Es hat sich im Hinblick auf die Steigerung der Ausbeute als besonders vorteilhaft erwiesen, wenn man für diesen Zweck verdünnte Basen einsetzt, die einen Puffer enthalten. Ein ausgezeichnetes System stellt verdünnte (z.B. 0,1 M) Natronlauge gepuffert mit (z.B. 0,3 M) Natriumchlorid dar.

Zusammenfassen beinhaltet das erfindungsgemäße Verfahren in einer bevorzugten Ausgestaltungsform die folgenden Schritte:
(a) Separation des Rahms aus Rohmilch bei einer Temperatur im Bereich von etwa 8 bis etwa 15 °C;
(b) Beladen einer Festkörper-Adsorptionssäule mit einem Adsorbens bestehend aus einem nicht-durchlässigen Material hoher Dichte und einem zur Proteinbindung befähigten lonenaustauscherharz;
(c) Versetzen des Adsorbens in der Säule in einen Wirbelschichtzustand;
(d) gegebenenfalls Spülen der Säule mit Wasser;
(e) Aufgeben der kalt separierten Magermilch aus Schritt (a);
(f) gegebenenfalls Spülen der Säule mit Wasser;
(g) Eluieren des Lactoperoxidase vom Adsorbens in der Säule mit verdünnter Base, die einen Puffer enthält;
(h) Entsalzen und Trocknen des Eluats zum Erhalt der Lactoperoxidase als trockenen Feststoff.

### ALLGEMEINE VORGEHENSWEISE FÜR DIE EBA

Eine EBA Säule mit einem Durchmesser von 2 cm wurde auf einen Magnetrührer platziert und mit 15 ml entmineralisiertem Wasser gefüllt. Die Säule wurde bis zu einer Höhe von etwa 20 cm mit Wolframperlen (Durchmesser ca. 200 µm) befüllt, die mit einem Sulfonsäurekationaustauscherharz der Firma UpFront Chromatography A/S, Dänemark (analog EP 1401289 B1, Beispiel 3) beschichtet waren. Der Magnetrührer wurde in Gang gesetzt und die Säule zunächst 15 Minuten mit entmineralisiertem Wasser bei einem Flux von etwa 10 cm/min gespült. Anschließen wurden verschiedene Milchfraktionen (Gewichtsverhältnis Adsorbens:Milchfraktion) etwa 1:200) aufgegeben und ein Flux von etwa 7,5 cm/min eingestellt. Nachdem die gesamte Menge der Milchfraktion die Säule passiert hatte, wurde diese wiederum 15 min mit entmineralisiertem Wasser gespült, ehe die Lactoperoxidase mit etwa 5 L einer verdünnten Natronlauge, bei einem Flux von etwa 7,5 cm/min eluiert wurde. Das Eluat wurde entsalzt und am Rotationsverdampfer vom Wasser befreit. Die Lactoperoxidase wurde anschließend als weißes trockenes Pulver erhalten.

### BEISPIEL 1

1.000 ml Rohmilch wurden bei 8 °C über einen Separator geleitet, wobei feste Bestandteile entfernt und der Rahm abgetrennt wurden. Die so erhaltene Magermilch wies einen natürlichen pH-Wert von 6,7 auf und wurde ohne weitere Behandlung auf die EBA-Säule aufgegeben. Es wurden 62 mg Lactoperoxidase in einer Reinheit von 93 % erhalten.

### BEISPIEL 2

Beispiel 1 wurde wiederholt, jedoch als Eluent eine 0,1 M Natronlauge eingesetzt, die 0,3 M Natriumchlorid als Puffer enthielt. Es wurden 69 mg Lactoperoxidase in einer Reinheit von 95 % erhalten.

### VERGLEICHSBEISPIEL V1

1.000 ml Rohmilch wurden ohne weiteren Separationsschritt auf die EBA-Säule aufgegeben. Das Säulenmaterial verklebte wegen des hohen Fettgehaltes innerhalb kurzer Zeit, so dass eine Elution praktisch nicht mehr möglich war.

### VERGLEICHSBEISPIEL V2

1.000 ml Rohmilch wurden bei etwa 50 °C über eine Kombination aus Plattenwärmeaustauscher und Separator geleitet, wobei feste Bestandteile entfernt und der Rahm abgetrennt wurden. Die so erhaltene Magermilch wies einen natürlichen pH-Wert von 6,7 auf und wurde anschließend bei 75 °C über einen Zeitraum von 30 Sekunden pasteurisiert und dann auf die EBA-Säule aufgegeben. Es wurden nur 49 mg Lactoperoxidase in einer Reinheit von 92 % erhalten.

### VERGLEICHSBEISPIEL V3

Vergleichsbeispiel V2 wurde wiederholt, jedoch als Eluent eine 0,1 M Natronlauge eingesetzt, die 0,3 M Natriumchlorid als Puffer enthielt. Es wurden 54 mg Lactoperoxidase in einer Reinheit von 93 % erhalten.

Die Beispiele und Vergleichsbeispiele zeigen, dass Rohmilch, die den höchsten Gehalt an Lactoperoxidase aufweist, für den Einsatz in chromatographischen Verfahren wegen des hohen Fettgehaltes ungeeignet ist. Magermilch, die nach dem konventionellen Verfahren der Warmseparation hergestellt wird, kann in die Chromatographie eingesetzt werden, liefert jedoch infolge der Wärmeinstabilität der Lactoperoxidase deutlich geringere Ausbeuten als Magermilch aus der Kaltseparation. Schließlich lässt sich durch die spezifische Auswahl des Eluenten Ausbeute und Reinheit an Lactoperoxidase signifikant beeinflussen.

## Patentansprüche

1. Verfahren zur Gewinnung von Lactoperoxidase, bei dem man
(a) Rohmilch einer Kaltseparation bei einer Temperatur von 8 bis 15 °C unterwirft und den Rahm abtrennt,
(b) die kalt separierte Magermilch einer Expanded Bed Adsorption (EBA) unterwirft, wobei die Lactperoxidase vom Adsorbens in der EBA-Säule adsorbiert wird, und anschließend
(c) die Lactoperoxidase wieder eluiert, entsalzt, entwässert und als trockenes Produkt isoliert.

2. Verfahren nach Anspruch 1, **dadurch gekennzeichnet, dass** man die Rohmilch bei einer Temperatur von 8 bis 12 °C entrahmt.

3. Verfahren nach den Ansprüchen 1 und/oder 2, **dadurch gekennzeichnet, dass** man eine Festbett-Adsorptionssäule einsetzt.

4. Verfahren nach mindestens einem der Ansprüche 1 bis 3, **dadurch gekennzeichnet, dass** man die EBA mit einem Adsorbens durchführt, dass aus einem nicht-durchlässigen Material hoher Dichte und einem zur Proteinbindung befähigten lonenaustauscherharz besteht.

5. Verfahren nach Anspruch 4, **dadurch gekennzeichnet, dass** das nicht-durchlässige Material eine Dichte von mindestens 1,5 g/ml und/oder eine Teilchengröße von höchstens 150 µm aufweist.

6. Verfahren nach Anspruch 5, **dadurch gekennzeichnet, dass** das nicht-durchlässige Material Glas- oder Metallperlen darstellt.

7. Verfahren nach Anspruch 4, **dadurch gekennzeichnet, dass** das lonenaustauscherharz ein Sulfonsäure-Anionenaustauscherharz ist.

8. Verfahren nach mindestens einem der Ansprüche 1 bis 7, **dadurch gekennzeichnet, dass** man die EBA-Säule vor und nach der Beladung mit der kalt separierten Magermilch spült.

9. Verfahren nach mindestens einem der Ansprüche 1 bis 7, **dadurch gekennzeichnet, dass** man die kalt separierte Magermilch mit einer Fluxrate von etwa 3 bis etwa 50 cm/min aufbringt.

10. Verfahren nach mindestens einem der Ansprüche 1 bis 9, **dadurch gekennzeichnet, dass** man das nicht-durchlässigen Material hoher Dichte und das zur Proteinbindung befähigten lonenaustauscherharz im Gewichtsverhältnis von etwa 20:80 bis etwa 50:50 einsetzt.

11. Verfahren nach mindestens einem der Ansprüche 1 bis 10, **dadurch gekennzeichnet, dass** man das Adsorbens bestehend aus dem nicht-durchlässigen Material hoher Dichte und dem zur Proteinbindung befähigten lonenaustauscherharz relativ zu dem auf die EBA-Säule zu ladenden die kalt separierten Magermilch in einem v/v-Verhältnis von etwa 1:100 bis etwa 1:1.000 einsetzt.

12. Verfahren nach mindestens einem der Ansprüche 1 bis 10, **dadurch gekennzeichnet, dass** man die Lactoperoxidase mit verdünnter Base eluiert.

13. Verfahren nach Anspruch 12, **dadurch gekennzeichnet, dass** man verdünnte Base einsetzt, die einen Puffer enthält.

14. Verfahren nach den Ansprüchen 12 und/oder 13, **dadurch gekennzeichnet, dass** man verdünnte Natronlauge gepuffert mit Natriumchlorid einsetzt.

15. Verfahren nach Anspruch 1 umfassend die folgenden Schritte:
(a) Separation des Rahms aus Rohmilch bei einer Temperatur im Bereich von etwa 8 bis etwa 15 °C;
(b) Beladen einer Festkörper-Adsorptionssäule mit einem Adsorbens bestehend aus einem nicht-durchlässigen Material hoher Dichte und einem zur Proteinbindung befähigten lonenaustauscherharz;
(c) Versetzen des Adsorbens in der Säule in einen Wirbelschichtzustand;
(d) gegebenenfalls Spülen der Säule mit Wasser;
(e) Aufgeben der kalt separierten Magermilch aus Schritt (a);
(f) gegebenenfalls Spülen der Säule mit Wasser;
(g) Eluieren des Lactoperoxidase vom Adsorbens in der Säule mit verdünnter Base, die einen Puffer enthält;
(h) Entsalzen und Trocknen des Eluats zum Erhalt der Lactoperoxidase als trockenen Feststoff.

## Claims

1. A method of recovering lactoperoxidase comprising:
(a) subjecting raw milk to a cold separation at a temperature of from 8 to 15 °C and separating the cream;
(b) subjecting the cold separated skimmed milk to Expanded Bed Adsorption (EBA), wherein the lactoperoxidase is adsorbed by the adsorbent in the EBA column; and then
(c) the lactoperoxidase is re-eluted, desalted, dehydrated and isolated as a dry product.

2. A method according to claim 1, **characterized in that** the raw milk is skimmed off at a temperature of from 8 to 12 °C.

3. Method according to claims 1 and/or 2, **characterized in that** a fixed bed adsorption column is used.

4. A method according to at least one of claims 1 to 3, **characterized in that** the EBA is carried out with an adsorbent consisting of a non-permeable material of high density and an ion exchange resin capable of protein binding.

5. A method according to claim 4, **characterized in that** the non-permeable material has a density of at least 1.5 g/ml and / or a particle size of at most 150 µm.

6. A method according to claim 5, **characterized in that** the non-permeable material is glass or metal beads.

7. A method according to claim 4, **characterized in that** the ion exchange resin is a sulfonic acid anion exchange resin.

8. Method according to at least one of claims 1 to 7, **characterized in that** the EBA column is rinsed with the cold-separated skimmed milk before and after the loading.

9. Process according to at least one of claims 1 to 7, **characterized in that** the cold-separated skimmed milk is applied at a flux rate of about 3 to about 50 cm/min.

10. A method according to any one of claims 1 to 9, **characterized in that** the non-permeable material of high density and the ion exchange resin capable of protein binding in a weight ratio of about 20:80 to about 50:50 is used.

11. Process according to at least one of Claims 1 to 10, **characterized in that** the adsorbent consisting of the non-permeable high-density material and the ion exchange resin capable of protein binding is applied relative to the cold-separated skimmed milk to be loaded onto the EBA column in a v/v ratio of about 1: 100 to about 1: 1000.

12. Process according to at least one of Claims 1 to 10, **characterized in that** the lactoperoxidase is eluted with dilute base.

13. A method according to claim 12, **characterized in that** a dilute base is used which contains a buffer.

14. Process according to claims 12 and/or 13, **characterized in that** dilute sodium hydroxide solution buffered with sodium chloride is used.

15. The process of claim 1 comprising the steps of:
(a) separating the cream from raw milk at a temperature in the range of about 8 to about 15 °C;
(b) loading a solid adsorption column with an adsorbent consisting of a non-permeable high density material and an ion exchange resin capable of protein binding;
(c) placing the adsorbent in the column in a fluidized bed state;
(d) optionally rinsing the column with water;
(e) applying the cold-separated skimmed milk from step (a);
(f) optionally rinsing the column with water;
(g) eluting the lactoperoxidase from the adsorbent in the column with dilute base, which contains a buffer;
(h) desalting and drying the eluate to obtain the lactoperoxidase as a dry solid.

## Revendications

1. Procédé d'obtention de lactoperoxydase, selon lequel
(a) du lait brut est soumis à une séparation à froid à une température de 8 à 15 °C et la crème est séparée,
(b) le lait écrémé séparé à froid est soumis à une adsorption en lit expansé (Expanded Bed Adsorption, EBA), la lactoperoxydase étant adsorbée par l'adsorbant dans la colonne d'EBA, puis
(c) la lactoperoxydase est de nouveau éluée, dessalée, déshydratée et isolée sous la forme d'un produit sec.

2. Procédé selon la revendication 1, **caractérisé en ce que** le lait brut est écrémé à une température de 8 à 12 °C.

3. Procédé selon les revendications 1 et/ou 2, **caractérisé en ce qu'**une colonne d'adsorption à lit fixe est utilisée.

4. Procédé selon au moins l'une quelconque des revendications 1 à 3, **caractérisé en ce que** l'EBA est réalisée avec un adsorbant qui est constitué par un matériau non perméable de densité élevée et une résine échangeuse d'ions apte à la liaison de protéines.

5. Procédé selon la revendication 4, **caractérisé en ce que** le matériau non perméable présente une densité d'au moins 1,5 g/ml et/ou une taille de particule d'au plus 150 µm.

6. Procédé selon la revendication 5, **caractérisé en ce que** le matériau non perméable consiste en des perles de verre ou de métal.

7. Procédé selon la revendication 4, **caractérisé en ce que** la résine échangeuse d'ions est une résine échangeuse d'anions acide sulfonique.

8. Procédé selon au moins l'une quelconque des revendications 1 à 7, **caractérisé en ce que** la colonne d'EBA est rincée avant et après le chargement avec le lait écrémé séparé à froid.

9. Procédé selon au moins l'une quelconque des revendications 1 à 7, **caractérisé en ce que** le lait écrémé séparé à froid est introduit en un débit d'environ 3 à environ 50 cm/minute.

10. Procédé selon au moins l'une quelconque des revendications 1 à 9, **caractérisé en ce que** le matériau non perméable de densité élevée et la résine échangeuse d'ions apte à la liaison de protéines sont utilisés en un rapport en poids d'environ 20:80 à environ 50:50.

11. Procédé selon au moins l'une quelconque des revendications 1 à 10, **caractérisé en ce que** l'adsorbant constitué par le matériau non perméable de densité élevée et la résine échangeuse d'ions apte à la liaison de protéines est utilisé par rapport au lait écrémé séparé à froid à charger dans la colonne d'EBA en un rapport v/v d'environ 1:100 à environ 1:1000.

12. Procédé selon au moins l'une quelconque des revendications 1 à 10, **caractérisé en ce que** la lactoperoxydase est éluée avec une base diluée.

13. Procédé selon la revendication 12, **caractérisé en ce qu'**une base diluée qui contient un tampon est utilisée.

14. Procédé selon les revendications 12 et/ou 13, **caractérisé en ce que** de la soude caustique diluée, tamponnée avec du chlorure de sodium, est utilisée.

15. Procédé selon la revendication 1, comprenant les étapes suivantes :
(a) la séparation de la crème du lait brut à une température dans la plage allant d'environ 8 à environ 15 °C ;
(b) le chargement d'une colonne d'adsorption à corps solides avec un adsorbant constitué par un matériau non perméable de densité élevée et une résine échangeuse d'ions apte à la liaison de protéines ;
(c) le mélange de l'adsorbant dans la colonne à un état de lit fluidisé ;
(d) éventuellement le rinçage de la colonne avec de l'eau ;
(e) l'introduction du lait écrémé séparé à froid de l'étape (a) ;
(f) éventuellement le rinçage de la colonne avec de l'eau ;
(g) l'élution de la lactoperoxydase de l'adsorbant dans la colonne avec une base diluée, qui contient un tampon ;
(h) le dessalage et le séchage de l'éluat pour obtenir la lactoperoxydase sous la forme d'un solide sec.
